(19) Europäisches Patentamt
European Patent Office
Office européen des brevets

(11) **EP 3 018 464 A1**

(12) **EUROPEAN PATENT APPLICATION**
published in accordance with Art. 153(4) EPC

(43) Date of publication:
**11.05.2016 Bulletin 2016/19**

(51) Int Cl.:
**G01N 1/00** (2006.01)

(21) Application number: **14819589.4**

(22) Date of filing: **01.07.2014**

(86) International application number:
**PCT/JP2014/067535**

(87) International publication number:
**WO 2015/002193 (08.01.2015 Gazette 2015/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **02.07.2013   JP 2013138606**
**10.03.2014   JP 2014046086**
**30.06.2014   JP 2014133768**

(71) Applicant: **FUJI-FILM Corporation**
**Minato-ku**
**Tokyo 106-8620 (JP)**

(72) Inventors:
• **NISHIO Tomonori**
**Ashigarakami-gun**
**Kanagawa 258-8538 (JP)**

• **KIMURA Toshihito**
**Ashigarakami-gun**
**Kanagawa 258-8538 (JP)**
• **KUNIYASU Toshiaki**
**Ashigarakami-gun**
**Kanagawa 258-8538 (JP)**
• **NISHIJIMA Kazuteru**
**Ashigarakami-gun**
**Kanagawa 258-8538 (JP)**

(74) Representative: **Hoffmann Eitle**
**Patent- und Rechtsanwälte PartmbB**
**Arabellastraße 30**
**81925 München (DE)**

(54) **ADAPTER FOR BLOOD SAMPLE DISPENSING, AND DISPENSING KIT AND NEEDLE KIT PROVIDED THEREWITH**

(57)   There is provided an adapter for blood sample dispensing which enables change of the presence and absence of a medicine to be mixed in a blood sample or the type of the medicine thereof while suppressing contact of blood with the air, during preparation of an examination sample, and a dispensing kit and a needle kit provided therewith. Dispensing is performed using an adapter 1 for blood sample dispensing which is mounted on a distal portion of a syringe and has a piping structure, in which the adapter has a fitting portion 3 fitted to the distal portion at one end, a nozzle portion 2 at the other end, and a flange portion 4 on an outer circumference surface, and in which the adapter 1 has a medicine 5, to be mixed in the blood sample S, on the inside of the nozzle portion 2.

FIG. 1

EP 3 018 464 A1

## Description

## BACKGROUND OF THE INVENTION

1. Field of the Invention

The present invention relates to an adapter, which is mounted on a syringe when dispensing a liquid substance such as blood, and a dispensing kit provided therewith.

2. Description of the Related Art

Blood which has been collected from a patient (including a human and another animal) is dispensed into a container such as Spitz, and is then subjected to treatment such as centrifugation. At this time, in some cases, an anticoagulant is mixed with blood in order to prevent coagulation of blood.

Examples of the method of mixing an anticoagulant with blood include: (1) a method of collecting blood using a vacuum blood collection tube in which an anticoagulant is sealed in advance, and then, mixing the collected blood with inversion (JP1983-38536A (JP-S58-38536A)); (2) a method of collecting blood using a syringe in which an anticoagulant is applied to the inner wall thereof, and then, mixing the collected blood with inversion (JP2001-224575A); and (3) a method of dispensing blood, after being collected, into an examination container (for example, Microtina trace blood collection tube manufactured by Becton, Dickinson and Company (registered trademark)) in which an anticoagulant is sealed, and mixing the dispensed blood with inversion.

## SUMMARY OF THE INVENTION

However, in the above-described method of (1), in some cases, when blood is collected from an infant or a small animal, it is impossible to collect enough blood for a specified collection amount of a vacuum blood collection tube. In addition, in the method of (2), in some cases, the anticoagulant is mixed with all of the collected blood, and therefore, such a sample cannot be used in other examinations. In this case, it is necessary to collect blood again using another syringe, which is an inefficient operation. In addition, increase in the number of times (in particular, the number of times of piercing with a needle) of collecting blood increases a burden on a patient (in particular, on an animal), which is not preferable.

In addition, in the above-described method of (3), blood comes into contact with the air when dispensing blood into the examination container from a syringe. Therefore, coagulation easily occurs, and there is a restriction on an operation in that dispensing of blood needs to be promptly performed and mixing blood with inversion needs to be promptly performed after the dispensing. At this time, in a case where the dispensing is performed by, for example, a person who is not familiar with the dispensing operation, coagulation may not be able to be effectively prevented.

The present invention has been made from the viewpoint of above-described problems, and an object of the present invention is to provide an adapter for blood sample dispensing which enables change of the presence and absence of a medicine to be mixed in a blood sample or the type of the medicine thereof during dispensing while suppressing contact of the blood sample with the air, and a dispensing kit and a needle kit provided therewith.

In order to solve the above-described problems, there is provided an adapter for blood sample dispensing which is mounted on a distal portion of a syringe and has a piping structure, in which the adapter has a fitting portion fitted to the distal portion of the syringe at one end, a nozzle portion at the other end, and a flange portion on an outer circumference surface, and in which the adapter has a medicine, to be mixed in the blood sample, on the inside of the nozzle portion.

Moreover, in the adapter of the present invention, it is preferable that the inner wall surface of the nozzle portion is a bumpy surface. In addition, it is preferable that the adapter has an inner wall projection portion, which is formed in a spiral shape, on the inner wall surface of the nozzle portion.

In addition, in the adapter of the present invention, it is possible to employ a structure in which the medicine is applied to the inner wall of the nozzle portion or a structure in which the medicine is supported by a support.

In addition, in the adapter of the present invention, it is preferable that an outer wall projection portion is erected on the outer wall surface of the nozzle portion, and a gap is caused between the flange portion and an opening portion by bringing the outer wall projection portion and a dispensing container into contact with each other when the nozzle portion is inserted into the opening portion which is formed in the dispensing container into which the blood sample is dispensed. In this case, it is preferable that the effective diameter of a distal portion of the nozzle portion, which is an effective diameter of the nozzle portion and a diameter of a minimum circumscribed circle of the nozzle portion and the outer wall projection portion, is a size allowing the distal portion to be inserted into the opening portion.

In addition, the outer wall projection portion has a tapered shape which tapers.

It is possible to employ a configuration in which the outer wall projection portion extends in an axial direction of the nozzle portion. In this case, it is preferable that the outer wall projection portion has a step portion in which the effective diameter of the nozzle portion changes from a value greater than the inner diameter of the opening portion to a value which allows the adapter to be fitted to the opening portion, toward a distal end. In addition, it is preferable that there are three or more outer wall projection portions which are disposed in parallel in a circumferential direction of the nozzle portion.

It is possible to employ a configuration in which the outer wall projection portion extends in a spiral shape along the circumferential surface of the nozzle portion.

In addition, in the adapter of the present invention, the length L between a distal end of the outer wall projection portion and the distal end of the nozzle portion satisfies the following Formula 1 when the inner diameter of the opening portion is set to W, the depth of the opening portion is set to D, the outer diameter of the distal end of the nozzle portion is set to $\phi1$, and the effective diameter of the nozzle portion in a section, in which a gap for the nozzle portion inclining in the opening due to the difference between the inner diameter of the opening portion and the effective diameter of the nozzle portion is formed between the opening portion and the section, out of sections of the nozzle portion and the outer wall projection portion, is set to $\phi2$.

$$\frac{\phi1}{2} \cdot \frac{W - \phi2}{D} \leq L < D \qquad \text{Formula 1}$$

In addition, in a case where the adapter of the present invention is provided with the outer wall projection portion, it is possible to employ a configuration in which the effective diameter of a distal portion of the nozzle portion is larger than the inner diameter of the opening portion.

In addition, in the adapter of the present invention, the flange portion may have a through port or a notch.

In addition, in the adapter of the present invention, it is preferable that the length of the nozzle portion is 3 mm to 30 mm and the outer diameter of the flange portion is 8 mm to 30 mm.

In addition, in the adapter of the present invention, it is preferable that the volume of an internal space of the nozzle portion is 10 $\mu$L to 1 mL.

In addition, in the adapter of the present invention, it is preferable that the adapter has a specific color corresponding to the type of the medicine.

A dispensing kit of the present invention includes: the adapter described above which is provided with the outer wall projection portion; and a dispensing container having an opening portion which is fitted to the outer wall projection portion.

Furthermore, a needle kit of the present invention includes: the adapter described above; and a needle having a needle base which is fitted to the distal portion of the syringe. The nozzle portion of the adapter also capable of being fitted to the needle base. Moreover, the needle kit of the present invention may also include the syringe having the distal portion which is fitted to the fitting portion of the adapter.

The adapter for blood sample dispensing of the present invention, and the dispensing kit and the needle kit provided wherewith include a medicine, which is to be mixed in a blood sample, on the inside of the nozzle portion. Therefore, it is possible to mix the medicine in the blood sample by simply mounting the adapter on the syringe for dispensing only when it is necessary to mix the medicine in the blood sample. As a result, due to the attachment/detachment of the adapter, it is possible to change the presence and absence of a medicine to be mixed in a blood sample or the type of the medicine thereof during dispensing while suppressing the contact of the blood sample with the air.

**BRIEF DESCRIPTION OF THE DRAWINGS**

Fig. 1 is a schematic view showing a configuration of an adapter according to a first embodiment.
Figs. 2A and 2B are a cross-sectional schematic view showing the adapter in a state of being mounted on a syringe.
Fig. 3 is a cross-sectional schematic view showing a state in which dispensing is performed using the adapter.
Fig. 4 is a cross-sectional schematic view showing a modification example of an adapter according to the first embodiment.
Fig. 5 is a cross-sectional schematic view showing an example of a nozzle portion having a structure causing a turbulent flow.
Fig. 6 is a cross-sectional schematic view showing an example of a nozzle portion having a structure causing a turbulent flow.
Fig. 7 is a cross-sectional schematic view showing a modification example of the adapter according to the first embodiment.
Figs. 8A and 8B are schematic views showing a configuration of an adapter according to a second embodiment.
Figs. 9A to 9D are schematic views showing a configuration of a dispensing kit according to the second embodiment.
Figs. 10A to 10D are schematic views showing other embodiments of adapters in order to secure a ventilation passage.
Figs. 11A to 11C are cross-sectional schematic views showing a configuration of a dispensing kit according to a third embodiment.
Figs. 12A and 12B are cross-sectional schematic views showing a configuration of an adapter of the third embodiment.
Figs. 13A to 13C are cross-sectional schematic views showing a dispensing step in which an adapter of which the distance between distal ends of a nozzle portion and an outer wall projection portion is too long is used.
Fig. 14 is a cross-sectional schematic view showing an adapter, of which the distance between distal ends of a nozzle portion and an outer wall projection portion is too short, and a sample which remains at the distal end of the nozzle portion.

Figs. 15A to 15D are cross-sectional schematic views showing a dispensing step in which the adapter according to the third embodiment is used.

Fig. 16 is a schematic view showing a state in which a modeled nozzle portion is inserted into an opening of a dispensing container.

Figs. 17A and 17B are cross-sectional schematic views showing the adapter according to the third embodiment and the sample remaining at the distal end of the nozzle portion.

Figs. 18A to 18C are cross-sectional schematic views showing a configuration of a needle kit according to a fourth embodiment.

## DESCRIPTION OF THE PREFERRED EMBODIMENTS

Hereinafter, embodiments of the present invention will be described using the drawings, but the present invention is not limited thereto. In order to allow easy visibility, the scale or the like of each of the components in the drawing is appropriately different from reality.

In the following embodiments, a syringe, which is used during blood collection and a blood examination, and an adapter and a dispensing container corresponding to the syringe will be described in order to provide clear explanation.

[First Embodiment]

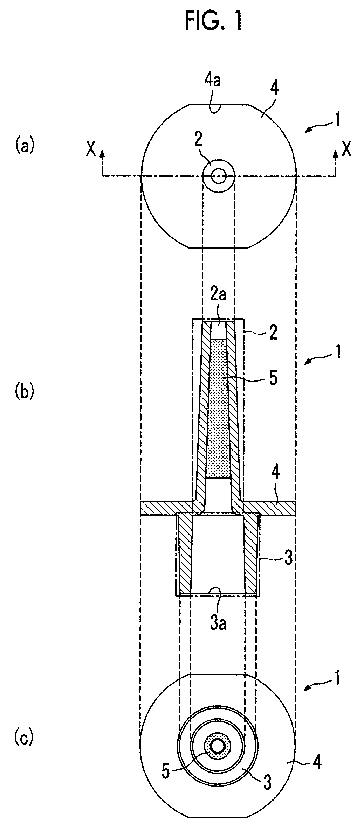

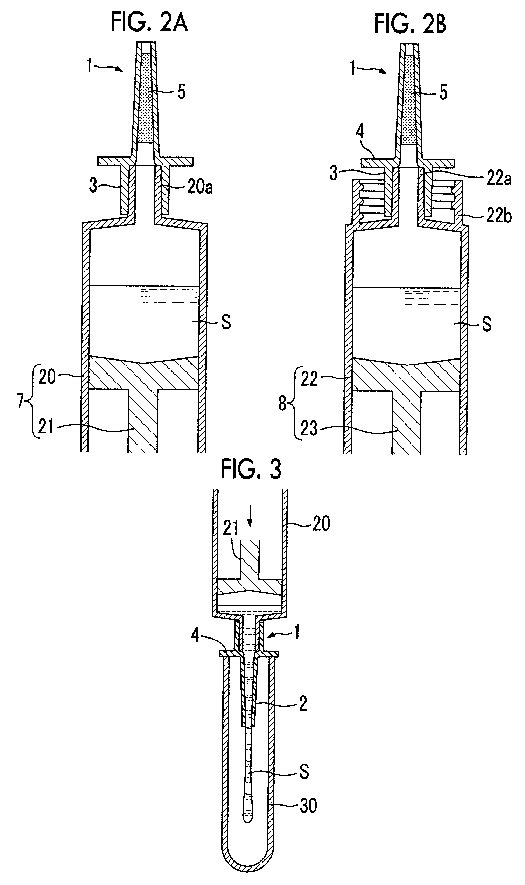

First, a first embodiment of an adapter will be described. Fig. 1 is a schematic view showing a configuration of the adapter according to the present embodiment. Specifically, (a) of Fig. 1 is a top view of the adapter, (b) of Fig. 1 is a cross-sectional view taken along line X-X in (a) of Fig. 1, and (c) of Fig. 1 is a bottom view. Figs. 2A and 2B are cross-sectional schematic views showing the adapter in a state of being mounted on a syringe. Fig. 3 is a cross-sectional schematic view showing a state in which dispensing is performed using the adapter.

As shown in Figs. 1 and 2, an adapter 1 for blood sample dispensing according to the present embodiment has a piping structure, of which one end is a fitting portion 3 fitted to a distal portion 20a of a syringe 7 (that is, a syringe main body 20) and the other end is a nozzle portion 2, and a flange portion 4 on an outer circumference surface of the piping structure. Moreover, in the adapter 1 according to the present embodiment, the inner wall which is the inside of the nozzle portion 2 is coated with an anticoagulant 5 (medicine) such as heparin which is to be mixed in blood.

In addition, the syringe kit according to the present embodiment is constituted of the adapter according to the present embodiment; and the syringe 7 which is formed of, for example, the syringe main body 20 and a plunger 21 as shown in Fig. 2A. The syringe may be a syringe 8 which is formed of a syringe main body 22, having a needle locking portion 22b around the distal portion 22a, and a plunger 23.

The nozzle portion 2 is a section of the piping structure which the distal portion 20a of the syringe 7 does not reach when the adapter 1 is mounted on the syringe 7. The inner wall surface of the nozzle portion 2 is coated with the anticoagulant 5. When blood S in the syringe 7 flows out from the nozzle portion 2, the blood S and the anticoagulant 5 come into contact with each other, and therefore, the anticoagulant 5 is mixed in the blood S. The outer diameter of a distal end 2a of the nozzle portion 2 is preferably smaller than the inner diameter (smallest inner diameter out of inner diameters of spots) of the fitting portion 3. The inner diameter of the fitting portion 3 reflects the outer diameter of the distal portion 20a of the syringe 7, and this is because the outer diameter of the distal end 2a of the nozzle portion 2 becomes smaller than that of the distal portion 20a as the outer diameter of the distal end 2a of the nozzle portion 2 is smaller than the inner diameter of the fitting portion 3. Accordingly, an operation of inserting the nozzle portion 2 into a container becomes easy. In addition, the inner diameter of the distal end 2a of the nozzle portion 2 is preferably greater than or equal to 0.5 mm in order to prevent hemolysis while blood passes the nozzle portion. The nozzle portion 2 preferably has a tapered shape which tapers toward the distal end.

Particularly, the length of the nozzle portion 2 is particularly preferably 3 mm to 30 mm. This is because the blood S and the anticoagulant 5 enter a state of being sufficiently mixed with each other (for example, in view of the amount of the anticoagulant 5 mixed in and evenness of the mixing) while the blood passes the nozzle portion if the length of the nozzle portion is longer than or equal to 3 mm, and handling of the adapter becomes easy during dispensing if the length of the nozzle portion is shorter than 30 mm. The length of the nozzle portion is the same length as that of a commercially available disposable chip for a pipette. In addition, the volume of the internal space of the nozzle portion 2 is preferably greater than or equal to 10 $\mu$L in view of not wasting a small amount of blood, and is preferably less than or equal to 1 mL in order to sufficiently mix blood with the anticoagulant.

The fitting portion 3 is a section of the piping structure which is fitted to the distal portion 20a of the syringe 7, and the shape, the inner diameter, and the length of the fitting portion, are designed to be suitable for the shape, the size, and the length of the distal portion 20a to be mounted on. For example, the fitting portion 3 is designed to be suitable for a syringe such as JIS T-3210 of the JIS standard. The adapter 1 is mounted on the syringe 7 through insertion of the distal portion 20a from an end portion 3a of the fitting portion 3 (refer to Fig. 2A). In the present embodiment, the fitting portion 3 and the distal portion 20a have a structure in which they are simply fitted to each other as shown in Fig. 2A. However, the fitting portion and the distal portion may have, for example, a screw structure or a structure in which they are engaged with each other using a convex section and a concave section (a recessed section or notched section).

The flange portion 4 has a function of preventing blood S from adhering to the hand when the adapter 1 is mounted on the syringe 7 using the hand, and a function of fixing the posture of the adapter 1 to a container 30 by bringing the adapter into contact with an opening portion of the container 30 as shown in Fig. 3. The outer diameter of the flange portion 4 is preferably 8 mm to 30 mm. The external shape of the flange portion 4 being greater than or equal to 8 mm reduces a concern that blood S may adhere to the hand and the fixation of the adapter with respect to the container 30 is stabilized. In contrast, if the outer diameter of the flange portion is less than or equal to 30 mm, the handling of the adapter 1 becomes easy. In addition, the flange portion 4 preferably has a rolling prevention portion 4a including a linear outer circumference portion, a notched portion, and the like. Accordingly, it is possible to prevent the rolling when the adapter 1 is disposed on a table. In the present embodiment, the flange portion 4 is formed all over the circumference of the piping structure, but it is not necessarily formed all over the circumference thereof. The position at which the flange portion 4 is formed in a direction along the center of the piping structure is not particularly limited. However, in view of facilitating attachment/detachment of the adapter 1, the flange portion is preferably positioned at a position at which the nozzle portion 2 and the fitting portion 3 are connected to each other, or the vicinity of the position. In addition, in a case where the adapter 1 is mounted on the distal portion 22a of the syringe 8 having the needle locking portion 22b as shown in Fig. 2B, the flange portion 4 is designed so as not to interfere with the needle locking portion 22b in advance.

The material of the adapter 1 is preferably a resin such as polyethylene (PE), polypropylene (PP), and polystyrene (PS). In addition, the adapter 1 is manufactured through, for example, integral molding using a resin as described above.

An examination using the adapter of the present invention is carried out as follows, for example. First, after collecting blood using a syringe, in a case of preparing an examination sample which is mixed with no anticoagulant, the blood in the syringe is dispensed into an examination container as it is without mounting an adapter. Then, after a required number of examination samples which are mixed with no anticoagulant are prepared, the process moves to preparation of examination samples which are mixed with an anticoagulant. In a case of preparing examination samples which are mixed with an anticoagulant, blood is dispensed into an examination container such as Spitz by mounting the adapter on the syringe. Then, the syringe and the adapter are discarded as they are after the completion of the preparation of all of the examination samples mixed with an anticoagulant. It is possible to easily prepare the examination samples which are mixed with an anticoagulant while suppressing the contact of blood with the air, by mounting the adapter on the syringe after the preparation of the examination samples which are mixed with no anticoagulant as described above. The same also applies to a case of examining a general blood sample (for example, a sample in which a medicine having no effect on all of examination results is mixed in blood in advance).

As described above, the adapter for blood sample dispensing according to the present embodiment includes an anticoagulant, which is to be mixed in blood, on the inside of the nozzle portion. Therefore, it is possible to mix the anticoagulant in blood by simply mounting the adapter on the syringe for dispensing only when it is necessary to mix the anticoagulant in blood. As a result, due to the attachment/detachment of the adapter, it is possible to change the presence and absence of an anticoagulant to be mixed in blood during dispensing while suppressing the contact of blood with the air.

<Modification Example according to First Embodiment>

In the present invention, the "medicine" refers to a compound to be mixed with blood. Accordingly, in the first embodiment, the case in which the medicine is an anticoagulant has been described. However, the medicine in the present invention is not limited thereto, and a coagulation accelerant or a separating agent (including a serum separating agent and a blood plasma separating agent) may be used. For example, as the medicine, it is possible to use at least one kind of ethylenediaminetetraacetic acid (EDTA), heparin sodium, heparin lithium, sodium citrate, trisodium citrate, fluoride, and potassium oxalate as an anticoagulant, or at least one kind of silica, thrombin, and diatomaceous earth as a coagulation accelerant. In addition, examples of the separating agent include a polyester gel.

In addition, in the first embodiment, the case in which the presence and absence of a medicine is changed has been described. However, the adapter of the present invention may be used in order to change the type of the medicine. For example, a dispensing method of performing dispensing using an adapter to which a type A medicine has been applied after first performing dispensing without adapter, and then, exchanging the adapter, to which the type A medicine has been applied, with an adapter to which a type B medicine has been applied for dispensing can be considered. Furthermore, in a case of applying different types of medicines as described above to adapters, if the colors of the adapters are set to specific colors corresponding to the types of the medicines, it becomes easy to visually recognize the types of the medicines, and therefore, it is possible to prevent a mistake. The color scheme complies with, for example, a color code of JIS T3233 of the JIS standard. The place to which color is applied may be the entire adapter or only a part of the adapter (for example, the flange portion, or an outer wall projection portion to be described below).

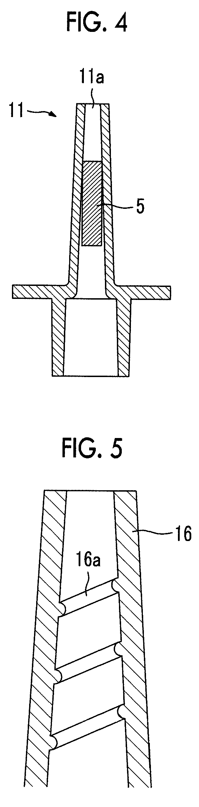

In addition, in the first embodiment, the case in which a medicine is applied to the inner wall of the nozzle portion has been described, but the present invention is not limited thereto. That is, in the present invention, provision of a medicine "on the inside of the nozzle portion" includes a case in which a medicine is held in an internal space of the nozzle portion as well as a case in which a medicine is applied only to the inner wall of the nozzle portion. For example, a support 15 (for example, a sheet formed of cotton or a non-woven fabric which can adsorb a medicine) supporting a medicine may be installed inside a nozzle portion 11 as shown in Fig. 4. The support 15 may be fixed to the inside of the nozzle portion 11 using another fixing member. However, it is not necessarily fixed depending on the shape of the nozzle portion 11. For example, if the inner diameter of a distal end 11a of the nozzle portion 11 is smaller than the width of the support 15, in general, there is no case where the support 15 comes out of the nozzle portion 11 during dispensing.

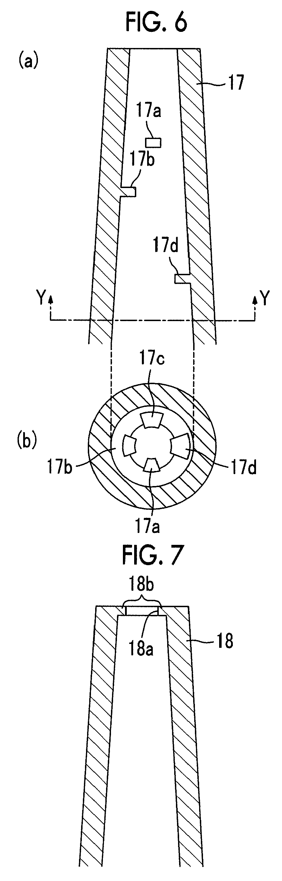

In addition, in the adapter of the present invention, a structure causing a turbulent flow may be formed on the inner wall of the nozzle portion in order to promote the mixing of a medicine with blood. For example, Fig. 5 is a view showing an example of a nozzle portion having a structure causing a turbulent flow. Specifically, in Fig. 5, a spiral-like inner wall projection portion 16a may be continuously formed on the inner wall of a nozzle portion 16. That is, because of the existence of the inner wall projection portion 16a, the flow of a blood sample is disturbed when the blood sample passes through the nozzle portion 16. In addition, Fig. 6 is a view showing another example of a nozzle portion having a structure causing a turbulent flow. (a) of Fig. 6 is a cross-sectional view passing through the center of a nozzle portion 17. (b) of Fig. 6 is a cross-sectional view taken along line Y-Y. Specifically, in Fig. 6, inner wall projection portions 17a, 17b, 17c, and 17d are formed on the inner wall of the nozzle portion 17 at predetermined intervals in a spiral shape. That is, because of the existence of the inner wall projection portions 17a, 17b, 17c, and 17d, the flow of a blood sample is disturbed when the blood sample passes through the nozzle portion 17. Alternately, the inner wall surface of the nozzle portion may be set to a bumpy surface on which a number of minute recesses and projections are formed. In this case, because of the existence of the minute recesses and projections, the flow of a blood sample is disturbed when the blood sample passes through the nozzle portion. Regarding the roughness due to the above-described recesses and projections on the bumpy surface of the inner wall surface, the ten-point average roughness which is measured through a method defined by JIS B0601 of the JIS standard is preferably 0.1 $\mu$m to 500 $\mu$m, more preferably 1 $\mu$m to 100 $\mu$m, and particularly preferably 5 $\mu$m to 50 $\mu$m. With the setting of the minute recesses and projections on the inner wall surface to have a shape within this range, the mixing of a blood sample and a medicine is favorably promoted.

In addition, in the adapter of the present invention, in order to secure difficulty of dripping of a medicine when the nozzle portion faces downward, a projection portion 18b protruding toward the center may be provided at an end portion 18a of a nozzle portion 18 as shown in Fig. 7.

[Second Embodiment]

Next, a second embodiment of an adapter will be described. The adapter according to the present embodiment is mainly different from the adapter according to the first embodiment in that the adapter according to the present embodiment has an outer wall projection portion on the outer wall surface of the nozzle portion. Accordingly, the detailed description of the same configuration as that in the first embodiment will not be repeated unless particularly necessary.

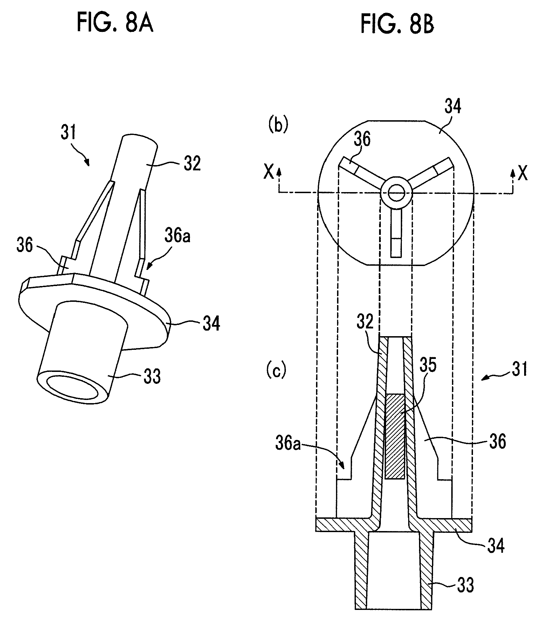

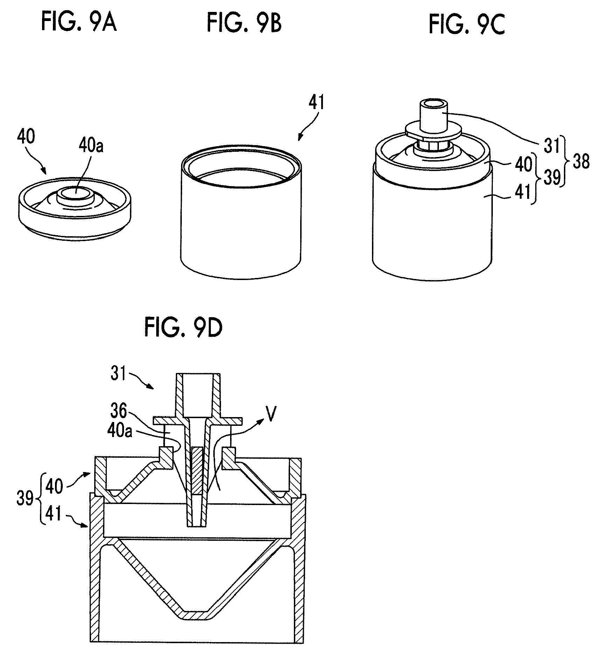

Figs. 8A and 8B are schematic views showing a configuration of an adapter 31 according to the present embodiment. Figs. 9A to 9D are schematic views showing a configuration of a dispensing kit 38 according to the present embodiment. Specifically, Fig. 8A is a perspective view of the adapter 31, (b) of Fig. 8B is an upper surface view of the adapter 31, and (c) of Fig. 8B is a cross-sectional view of the adapter 31 taken along line X-X of (b) of Fig. 8B. In addition, Fig. 9A is a perspective view of a lid 40 of a dispensing container 39 and Fig. 9B is a perspective view of a main body 41 of the dispensing container 39. Furthermore, Fig. 9C is a perspective view of the dispensing kit 38 when the adapter 31 is inserted into the dispensing container 39 and Fig. 9D is a cross-sectional view of the dispensing kit. The adapter 31 and the dispensing kit 38 are used by being favorably combined with each other in this manner.

As shown in Figs. 8A and 8B, the adapter 31 according to the present embodiment has a piping structure, of which one end is a fitting portion 33 fitted to a distal portion of a syringe, the other end is a nozzle portion 32, and the outer circumference surface is provided with a flange portion 34 and an outer wall projection portion 36. Moreover, in the adapter 31 according to the present embodiment, a support 35 supporting a medicine to be mixed in blood is installed in an internal space of the nozzle portion 32. In addition, the dispensing kit 38 according to the present embodiment is constituted of the dispensing container 39 and the adapter 31, and the dispensing container 39 is constituted of the lid 40 and the main body 41. The lid 40 has an opening 40a as an opening portion for dispensing a sample. For example, the opening 40a is formed of a cylindrical projection. For example, the dispensing container 39 is also used as a centrifugal container.

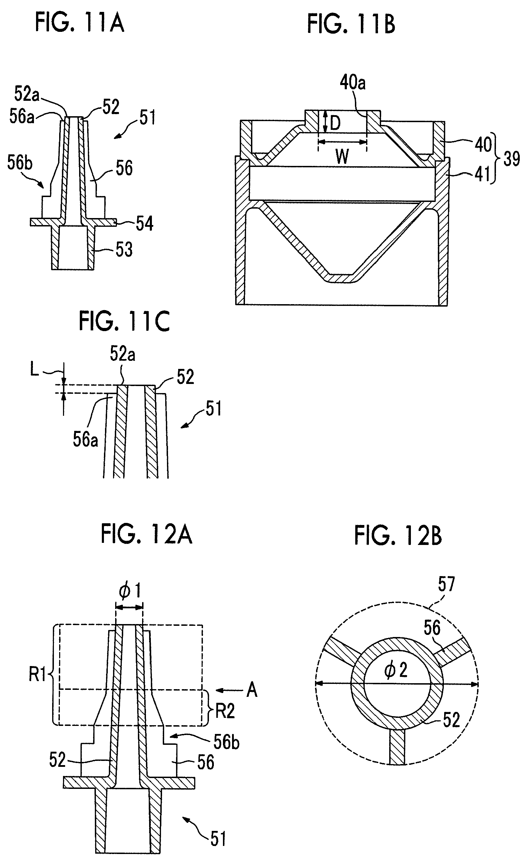

The outer wall projection portion 36 is constituted of three ribs (straight ribs) extending straight in an axial direction (direction along the central axis of the nozzle portion 32) of the nozzle portion 32 from the flange portion 34. The three ribs are erected at even intervals in a circumferential direction of the nozzle portion. In the outer wall projection portion 36, at least a part of the effective diameter of the nozzle portion on at least a distal side is set to a size such that the part of the outer wall projection portion is inserted into the opening 40a. The effective diameter of the nozzle portion at a certain position means an overall thickness (outer diameter) of the nozzle portion considering the size of the outer wall projection portion as well (that is, considering the existence of the outer wall projection portion), and is set to a diameter (refer to Fig. 12B to be described below) of a minimum circumscribed circle including the nozzle portion and the outer wall projection portion at the position. With the existence of such an outer wall projection portion 36, when the adapter 31 is inserted into the dispensing container 39 as shown in Figs. 9C and 9D, a gap which becomes a passage for air (ventilation passage V) is formed in the opening 40a. Accordingly, it is possible to prevent the increase in pressure on the inside of the container in accordance with dispensing. Therefore, it is possible to smoothly perform a dispensing operation. In addition, the adapter 31 is fixed to the dispensing container 39 using the outer wall projection portion 36 fitted to the opening 40a of the dispensing container 39, and therefore, it is possible to stably perform the dispensing operation. The outer wall projection portion 36 may be formed of the same material as those of the portions other than the outer wall projection portion of the adapter 31, or may be formed of a different material therefrom. In a case where the outer wall projection portion is formed of the same material thereas, it is possible to produce the outer wall projection portion through, for example, integral molding. In addition, in a case where the outer wall projection portion is formed of a different material therefrom, a method can be employed in which an adapter without an outer wall projection portion is produced, and then, the outer wall projection portion 36 is attached to the adapter. For example, the adhesiveness between the outer wall projection portion 36 and the dispensing container 39 increases by employing a material, which has higher elasticity than the material of the adapter 31, as the outer wall projection portion, and therefore, it is possible to more stably perform the dispensing operation.

The outer wall projection portion 36 preferably has a tapered shape which tapers as shown in Figs. 8A and 8B. Accordingly, the outer wall projection portion 36 also functions as an insertion guide when the adapter 31 is inserted into the dispensing container 39, thereby facilitating the insertion operation. In addition, with the provision of the tapered shape, the outer wall projection portion 36 functions as an insertion stopper with respect to the opening 40a of the dispensing container 39 which has various sizes. Therefore, the dispensing container is not restricted to have an opening with a specific size, and the range of the dispensing container which can be combined with the adapter 31 is widened. If the diameter of the circle which is circumscribed to the outer wall projection portion and the size of the opening of the dispensing container are appropriately designed so as to be fitted to each other, the outer wall projection portion may not necessarily have the tapered shape. Furthermore, as shown in Figs. 8A and 8B, the outer wall projection portion 36 preferably has a step portion 36a in which the effective diameter of the nozzle portion 32 changes from a value greater than the size of the above-described opening 40a to a value which allows the adapter 31 to be fitted to the above-described opening 40a, toward the distal end. With the provision of the above-described step portion 36a, the stability of the dispensing container 39 which has an opening with a specific size and is fitted to the step portion 36a is improved when the adapter 31 is inserted into the dispensing container and fixed thereto.

As described above, the adapter for blood sample dispensing according to the present embodiment and the dispensing kit provided therewith include a medicine, to be mixed in a blood sample, on the inside of the nozzle portion, and therefore, the same effect as that of the first embodiment is exhibited. In addition, the adapter according to the present embodiment includes the outer wall projection portion which forms a ventilation passage on the outer wall surface of the nozzle portion when the nozzle portion is inserted into the opening of the dispensing container, and therefore, it is possible to smoothly perform the dispensing operation.

<Modification Example according to Second Embodiment>

In the second embodiment, the case in which a medicine is supported by a support has been described. However, needless to say, a medicine in the present embodiment may also be applied to the inner wall of the nozzle portion 32. In addition, the ribs constituting the outer wall projection portion in the present invention is not limited to the three straight ribs. That is, the number of ribs may be one, two, or greater than or equal to four. Alternately, the ribs may extend so as to be twisted in an axial direction of the nozzle portion. However, the number of ribs is preferably greater than or equal to 3 in view of safety when an adapter is inserted into a dispensing container. In addition, in a case where there are a plurality of ribs, it is unnecessary for the plurality of ribs to be arranged at even intervals. In addition, the plate-like ribs have been described in the present embodiment. However, the shapes of the ribs are not limited thereto, and may be, for example, a triangular pyramid. Furthermore, it is unnecessary for the ribs to be connected to the flange portion.

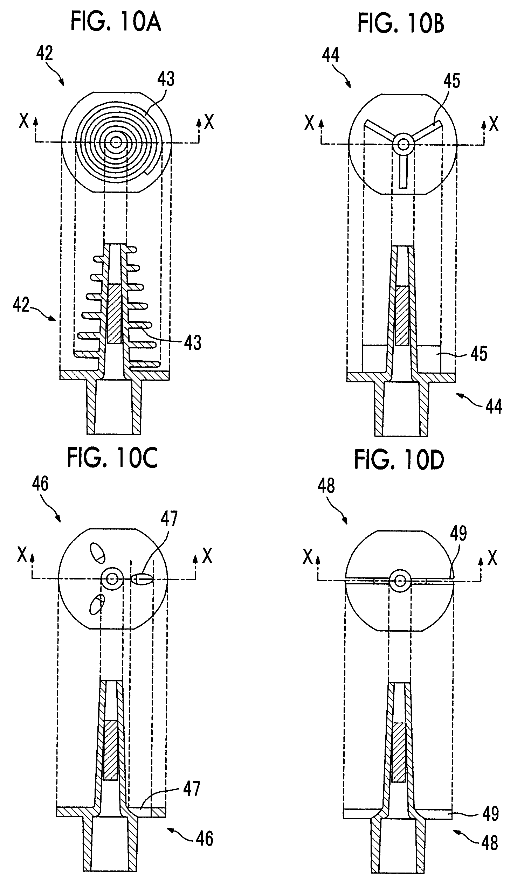

Furthermore, Figs. 10A to 10D are schematic views showing other modification examples of adapters in order to secure a ventilation passage. Specifically, Fig. 10A is a top view and a cross-sectional view of an adapter 42. The adapter 42 has an outer wall projection portion 43 constituted of a rib (spiral rib) extending in a spiral shape along the circumferential surface of the nozzle portion in a circumferential direction. In this case, the ventilation passage is formed in a spiral shape between ribs. In addition, this spiral rib has a tapered shape which tapers. Accordingly, an opening and the spiral rib come into contact with each other at an adequate position when the adapter 42 is inserted into a dispensing container. Therefore, it is possible to fix the adapter 42 to the dispensing container and to make the adapter correspond to dispensing containers which have openings with various sizes. Furthermore, in the case of an outer wall projection portion 43, since the ventilation passage is formed in a spiral shape, there is an advantage in that it is possible to prevent a sample from scattering during dispensing. The opening of the dispensing container into which the adapter 42 is inserted may have or may not have a structure in which the above-described spiral rib is screwed into the inner wall of the opening (that is, a fitting structure in a screwy manner). In a case where the adapter 42 and the dispensing container are screwed in this manner, a cut which vertically divides the spiral rib may be made in order to improve the circulation of the air.

In addition, Fig. 10B is a top view and a cross-sectional view of an adapter 44. The adapter 44 has an outer wall projection portion 45 formed of three rectangular ribs which have been arranged in the periphery of the nozzle portion. In the case of the outer wall projection portion 45, since the effective diameter (diameter of a minimum circle circumscribed on the outer wall projection portion 45) of a distal portion of the nozzle portion is designed to be larger than the inner diameter of the opening portion (diameter of the opening) of the dispensing container, the ribs do not advance from the opening, but it is possible to secure the ventilation passage. Even in this case, the ventilation passage is formed between the ribs.

In addition, Fig. 10C is a top view and a cross-sectional view of an adapter 46. The adapter 46 has a through port 47 in the flange portion. Accordingly, even if the adapter 46 is inserted into the dispensing container until the flange portion comes into contact with the opening portion of the dispensing container, it is possible to secure a ventilation passage using this through port 47. The through port 47 corresponds to a dispensing container with a small opening, and therefore, is preferably formed on the inner circumferential side of the flange portion.

In addition, Fig. 10D is a top view and a cross-sectional view of an adapter 48. The adapter 48 has a slit-like notch 49 crossing the flange portion except for the portion of the piping structure. Accordingly, even if the adapter 48 is inserted into the dispensing container until the flange portion comes into contact with the opening portion of the dispensing container, it is possible to secure a ventilation passage using this notch 49.

[Third Embodiment]

Next, a third embodiment of an adapter will be described. An adapter 51 according to the present embodiment is mainly different from the adapter according to the second embodiment in that the length L between a distal end 56a of an outer wall projection portion 56 and a distal end 52a of a nozzle portion 52 is set to be within a predetermined range. Accordingly, the detailed description of the same configuration as that in the second embodiment will not be repeated unless particularly necessary.

Figs. 11A to 11C are cross-sectional schematic views showing a configuration of a dispensing kit according to the third embodiment and Figs. 12A and 12B are cross-sectional schematic views showing a configuration of the adapter of the third embodiment. Specifically, Fig. 11A and Fig. 12A are cross-sectional views of the adapter according to the present embodiment and Fig. 11B is a cross-sectional view of a dispensing container according to the present embodiment. Moreover, Fig. 11C is an enlarged cross-sectional view of a distal portion of the adapter according to the present embodiment. In addition, Fig. 12B is an end surface view of a cut portion viewed from the top when the nozzle portion is sectioned at a position A in Fig. 12A.

As shown in Figs. 11 and 12, the adapter 51 according to the present embodiment has a piping structure, of which one end is a fitting portion 53 fitted to a distal portion of a syringe, the other end is a nozzle portion 52, and the outer circumference surface is provided with a flange portion 54 and an outer wall projection portion 56. Moreover, even in the present embodiment, a support (not shown in the drawing) supporting a medicine to be mixed in blood is installed in an internal space of the nozzle portion 52. In addition, the dispensing kit according to the present embodiment is constituted of the adapter 51 and the dispensing container 39. The dispensing container 39 is the same as the container described in the second embodiment and has the opening 40a into which the adapter 51 is inserted.

Similarly to the second embodiment, the outer wall projection portion 56 is constituted of three ribs (straight ribs) extending straight in an axial direction of the nozzle portion 52 from the flange portion 54. The three ribs are erected at even intervals in a circumferential direction of the nozzle portion. In addition, the outer wall projection portion 56 has a step portion 56b in which the effective diameter of the nozzle portion 52 changes from a value greater than the size of the above-described opening 40a to a value which allows the adapter 51 to be fitted to the above-described opening 40a, toward the distal end. Furthermore, the outer wall projection portion 56 has a tapering portion, in which the effective diameter of the nozzle portion 52 decreases toward the distal end of the nozzle portion 52, and extends up to the vicinity of the distal end 52a of the nozzle portion 52 while maintaining the effective diameter at the position at which the tapering ends, even after the position at which the tapering portion ends. The effective diameter of the nozzle portion 52 at a certain position is a diameter of a minimum circumscribed circle 57 including the nozzle portion 52 and the outer wall projection portion 56 at the position as described above.

The length L between the distal end 56a of the outer wall projection portion 56 and the distal end 52a of the nozzle portion 52 (which is a distance between the distal ends along an central axis of the nozzle portion 52; refer to Fig. 11C) is set to be within a predetermined range which is defined by the following Formula 2. In the following Formula 2, W represents an inner diameter of the opening portion (refer to Fig. 11B), D represents a depth of the opening portion (refer to Fig. 11B), and $\phi1$ represents an outer diameter of the distal end 52a of the nozzle portion 52 (refer to Fig. 12A). In addition, $\phi2$ represents an effective diameter of the nozzle portion 52 in a section R1, in which a gap for the nozzle portion 52 inclining in the opening 40a due to the difference between the size (inner diameter of the opening portion) of the opening 40a and the effective diameter of the nozzle portion 52 is formed between the opening portion and the section, out of sections of the nozzle portion 52 and the outer wall projection portion 56 (refer to Fig. 12A). In other words, the section R1 can also be called a section excluding a section (section in which the effective diameter and the opening diameter correspond to each other) fitted to the opening 40a, out of sections of the nozzle portion 52 and the outer wall projection portion 56 inserted into the opening 40a. In a case where there are a plurality of outer wall projection portions, it is particularly preferable that all of the outer wall projection portions satisfy the following Formula 2, but only a part of the outer wall projection portions may satisfy the following Formula 2.

$$\frac{\phi1}{2} \cdot \frac{W - \phi2}{D} \leq L < D \qquad \text{Formula 2}$$

In a case where the effective diameter $\phi2$ of the nozzle portion of the above-described section R1 is constant along an axial direction of the nozzle portion, the effective diameter is set to a constant value thereof, and in a case where the effective diameter changes along the axial direction of the nozzle portion, the effective diameter is set to a minimum effective diameter in a region R2 which is one-third of the above-described section R1 on the flange portion side. The reason that the region R2 which is one-third of the section R1 on the flange portion side is considered when the effective diameter changes along the axial direction of the nozzle portion is that, in many cases, in an operation in which the adapter is pulled out of the opening from a state (fitted state) in which the adapter is fitted to the opening, the nozzle portion is inclined in an initial stage of the pulling operation. The reason that there are many cases in which the nozzle portion is inclined in the initial stage of the pulling operation is that the adapter is swung from side to side when, for example, releasing the fitted state of the adapter, or the adapter is swung in order to drop blood remaining at a distal end of the nozzle portion within a dispensing container before the adapter is removed after the fitted state is released.

Accordingly, in the present embodiment, the effective diameter of the nozzle portion 52 in a terminal position A on the distal side of the region R2 in Fig. 12A is set to $\phi2$ (refer to Fig. 12B).

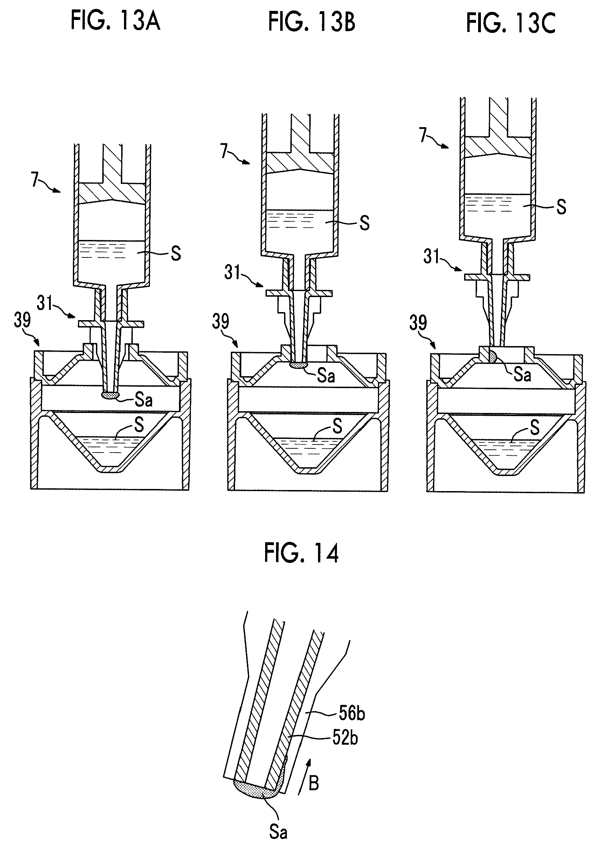

The upper limit of the above-described Formula 2 is based on the following reason. Figs. 13A to 13C are cross-sectional schematic views showing a dispensing step in which an adapter (for example, the adapter 31 shown in Figs. 8A and 8B) of which the distance L between distal ends of a nozzle portion and an outer wall projection portion is too long, that is, the distance L is greater than or equal to the depth D of an opening portion, is used. In general, the entire blood inside the syringe 7 is not dispensed into one dispensing container 39, and blood dispensed thereinto is a part of blood existing in the syringe 7. Accordingly, when dispensing of a necessary amount of blood S is finished, excessive blood Sa remains at the distal end of the nozzle portion (refer to Fig. 13A). In such a case, if the above-described distance L is greater than or equal to the depth D of the opening portion, the interference of the outer wall projection portion is avoided and the inner wall of the opening portion and the distal end of the nozzle portion approach each other when pulling the adapter 31 from the opening portion (refer to Fig. 13B). As a result, there is a possibility that blood Sa may adhere to the inner wall of the opening or the surface, such as a top surface, of the dispensing container when pulling the adapter 31 from the opening portion (refer to Fig. 13C). Accordingly, the above-described distance L is preferably less than the depth D of the opening portion in order to prevent the inner wall of the opening portion and the distal end of the nozzle portion from approaching each other.

In contrast, the lower limit of the above-described Formula 2 is based on the following reason. Fig. 14 is a cross-sectional schematic view showing an adapter, of which the distance between distal ends of a nozzle portion 52b and an outer wall projection portion 56b, is too short, that is, the distance L is less than $(\phi1/2) \cdot (W-\phi2/D)$, and a sample which remains at the distal end of the nozzle portion. For example, in Fig. 14, L is 0. As shown in Fig. 14, if the distance L is too short, in some cases, blood Sa remaining at the distal end of the nozzle portion 52b goes around the circumferential surface of the distal end of the nozzle portion 52b when the adapter is inclined. In this case, there is a possibility that the blood Sa which has gone around the above-described circumferential surface may be drawn in a direction B on the flange portion side along the gap between the nozzle portion 52b and the outer wall projection portion 56b due to a capillary phenomenon and be adhered to the surface of the adapter or the outer wall projection portion. Furthermore, if the blood Sa adheres to the surface of the adapter and the outer wall projection portion, there is a possibility that the blood Sa may be adhered to the inner wall of the opening or the surface, such as a top surface, of the dispensing container when pulling the adapter from the dispensing container and the blood Sa may be scattered in the periphery thereof through the dispensing operation. Therefore, the distal end 56a of the outer wall projection portion 56 is separated from the distal end of the nozzle portion so as not come into contact with the blood Sa which has gone around the circumferential surface of the distal end of the nozzle portion as described above.

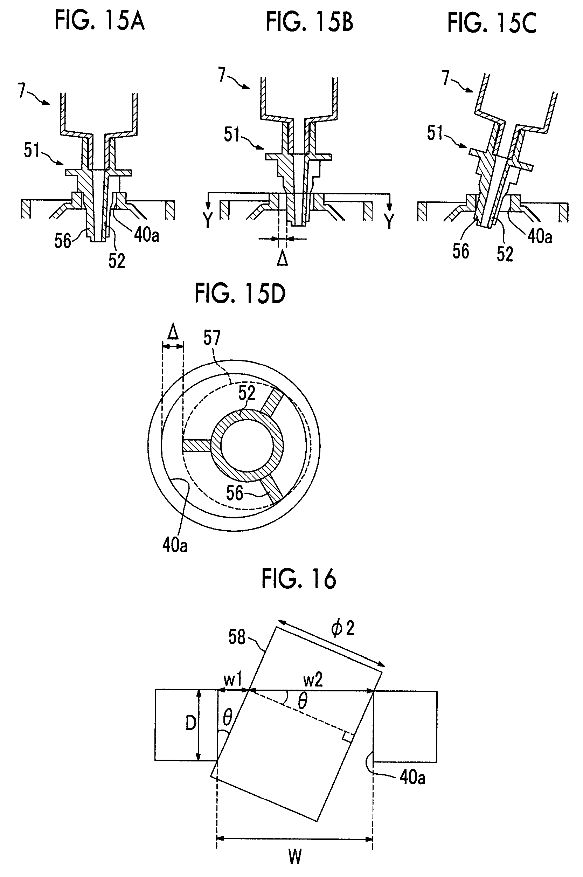

A specific lower limit value is determined through the following method. Figs. 15A to 15D are cross-sectional schematic views showing a dispensing step in which the adapter 51 according to the present embodiment is used. The gap Δ (maximum gap) for the nozzle portion 52 to be inclined within the opening 40a is caused (refer to Fig. 15B) when a state in which the adapter 51 is fitted to the opening 40a (refer to Fig.15A) is released after the adapter 51 is slightly pulled out of the opening 40a from the fitted state. Fig. 15D is an end surface view of a cut portion taken along line Y-Y of Fig. 15B. As can be seen from Fig. 15D, the size of the gap Δ in the state of Fig. 15B is almost the same as the size in which the effective diameter of the nozzle portion 52 is subtracted from the size (inner diameter of the opening portion) of the opening 40a. This gap Δ becomes so-called "play" of the nozzle portion 52 in the opening 40a, and the nozzle portion 52 can be inclined within the opening 40a (refer to Fig. 15C). Fig. 16 is a schematic view showing a state in which the modeled nozzle portion 52 is inserted into an opening of a dispensing container 39. In Fig. 16, the effective diameter of the nozzle portion 52 in the vicinity of the opening 40a is constant or approximated, and the nozzle portion 52 is modeled to a column 58 having a diameter $\phi2$. From this schematic view, a relationship which is represented by the following Formula 3 between W, D, $\phi2$, and $\theta$ is obtained.

$$W = w1 + w2$$
$$= D\tan\theta + \frac{\phi2}{\cos\theta} \quad \text{Formula 3}$$

In the above-described Formula 3, if $\theta$ is approximated to $\cos\theta \approx 1$ as a small value, the following Formula 4 can be obtained.

$$\tan\theta = \frac{W - \phi2}{D} \quad \text{Formula 4}$$

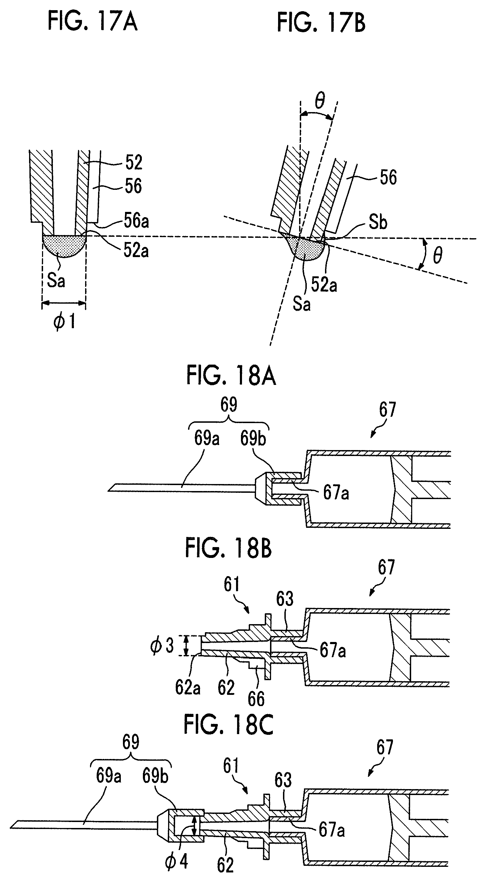

In contrast, in a case where the nozzle portion 52 is inclined at an angle $\theta$ as shown in Fig. 17B from a state in which the central axis of the nozzle portion 52 follows along a vertical direction as shown in Fig. 17A, blood Sa remaining at the distal end 52a of the nozzle portion 52 goes around the circumferential surface of the nozzle portion 52 by $(\phi1/2)\cdot\tan\theta$ in an axial direction. If the above-described Formula 4 is applied to $\tan\theta$ in the Formula, the lower limit of the above-described Formula 2 can be obtained.

For example, the inner diameter W of the opening portion of a general dispensing container is 6 mm and the depth D thereof is 2.5 mm. In the adapter corresponding to the above-described dispensing container, the outer diameter $\phi1$ of the distal end of the nozzle portion is 2.4 mm and the effective diameter $\phi2$ of the nozzle portion of the section in which the above-described gap is formed is 5.2 m. In this case, in the above-described calculation, the nozzle portion is inclined about 18 degrees, and the distance L between the distal end of the outer wall projection portion and the distal end of the nozzle portion is set to be within a range of greater than or equal to 0.8 mm and less than 2.5 mm.

As described above, the adapter for blood sample dispensing according to the present embodiment and the dispensing kit provided therewith include a medicine, to be mixed in a blood sample, on the inside of the nozzle portion, and therefore, the same effect as that of the first embodiment is exhibited. In addition, the adapter according to the present embodiment includes the outer wall projection portion which forms a ventilation passage on the outer wall surface of the nozzle portion when the nozzle portion is inserted into the opening of the dispensing container, and therefore, the same effect as that of the second embodiment is exhibited. Furthermore, in the adapter according to the present embodiment, the distance between the distal end of the outer wall projection portion and the distal end of the nozzle portion is set to be within the above-described predetermined range, and therefore, it is possible to reduce the phenomenon of adhesion of blood remaining at the distal end of the nozzle portion to the adapter and the dispensing container.

<Modification Example according to Third Embodiment>

In the third embodiment, the case in which the outer wall projection portion is constituted of the straight ribs has been described, but the outer wall projection portion may be formed of spiral ribs. That is, the distance between a distal end of a spiral rib and the distal end of the nozzle portion is set to be within the above-described predetermined range.

In the third embodiment, the case in which a medicine is supported by the support has been described. However, needless to say, the medicine may be applied to the inner wall of the nozzle portion 52. In addition, the ribs constituting the outer wall projection portion in the present invention are not limited to the three straight ribs, and the description of the ribs is the same as that in the second embodiment.

[Fourth Embodiment]

Next, a fourth embodiment of an adapter will be described. An adapter 61 according to the present embodiment is mainly different from the adapter according to the third embodiment in that a distal end 62a of a nozzle portion 62 can be fitted to a needle base 69b of a needle 69 which is attached to a syringe. Accordingly, the detailed description of the same configuration as that in the third embodiment will not be repeated unless particularly necessary.

Figs. 18A to 18C are cross-sectional schematic views showing a configuration of a needle kit according to the fourth embodiment. Specifically, Fig. 18A represents a state in which a syringe 67 and the needle 69 are fitted to each other and Fig. 18B represents a state in which the syringe 67 and the adapter 61 are fitted to each other. In addition, Fig. 18C represents a state in which the syringe 67 and the adapter 61 are fitted to each other and the needle 69 is fitted to the distal end 62a of the nozzle portion 62 of the adapter 61.

The needle kit according to the present embodiment is constituted of the adapter 61, the syringe 67, and the needle 69 as shown in Figs. 18A to 18C. In a case where the syringe 67 is to be separately prepared, the needle kit may be constituted of the adapter 61 and the needle 69. The syringe 67 is not particularly limited, and is, for example, the same as that in Fig. 2A.

The needle 69 is constituted of a needle main body 69a (needle pipe) and the needle base 69b which holds the needle main body 69a. The material or the size of the needle main body 69a is not particularly limited. For example, the material of the needle main body 69a is stainless alloy and the diameter of the pipe thereof is 0.4 mm to 1.2 mm. The material of the needle base 69b is, for example, resins such as PE, PP, and PS, and the needle base 69b has an opening portion which can be fitted to a distal portion 67a of the syringe 67. The needle 69 is used such that a fitting portion of the needle base 69b and the distal portion 67a of the syringe 67 are fitted to each other (refer to Fig. 18A) when, for example, collecting blood. After the collection of blood is finished, the needle 69 is removed from the syringe 67.

The adapter 61 is the same as, for example, the adapter 51 (refer to Fig. 11A) according to the third embodiment, but is different from the adapter 51 in that the outer diameter $\phi 3$ of the distal end 62a of the nozzle portion 62 is a size so as to be fitted to the fitting portion of the needle base 69b. That is, the outer diameter $\phi 3$ (including no outer wall projection portion 66) of the distal end 62a of the nozzle portion 62 coincides with the inner diameter $\phi 4$ of the fitting portion of the needle base 69b so as to be fitted to the inner diameter $\phi 4$ thereof. That is, in the present embodiment, the outer diameter $\phi 3$ of the distal end 62a of the nozzle portion 62, the inner diameter $\phi 4$ of the fitting portion of the needle base 69b, the inner diameter of a fitting portion 63, and the outer diameter of the distal portion 67a of the syringe 67 coincide with each other so as to be fitted to each other. The adapter 61 is used such that the fitting portion 63 of the adapter 61 and the distal portion 67a of the syringe 67 are fitted to each other (refer to Fig. 18B) when collecting blood is finished and the collected blood is dispensed after the needle 69 is removed from the syringe 67.

In the related art, when collection of blood is finished, the needle 69 is removed from the syringe 67 and is discarded as it is. In addition, when the dispensing is finished, the adapter 61 and the syringe 67 are discarded as they are in a state in which the adapter and the syringe are fitted to each other. However, if the needle kit according to the present embodiment is used, it is possible to integrally discard the needle 69, the adapter 61, and the syringe 67 in a state in which the needle 69 and the adapter 61 are fitted to each other and the adapter 61 and the syringe 67 are fitted to each other as shown in Fig. 18C. Accordingly, it is possible to close the opening of the nozzle portion 62 of the adapter 61 and the opening of the needle base 69b, and therefore, it is possible to prevent blood from scattering more than in the related art.

[Modification Example according to Fourth Embodiment]

In the fourth embodiment, the case in which the section of the distal end 62a of the nozzle portion 62 in which no outer wall projection portion 66 exists, and the needle base 69b can be fitted to each other has been described. However, a section of the distal end 62a of the nozzle portion 62 in which the outer wall projection portion 66 exists, and the needle base 69b may be fitted to each other. That is, in this case, the effective diameter of the nozzle portion 62 in which the outer wall projection portion 66 exists is set to a value which coincides with the inner diameter $\phi 4$ of the fitting portion of the needle base 69b so as to be fitted thereto.

Explanation of References

    1, 31, 42, 44, 46, 48, 51, 61: adapter
    2, 11, 32, 52, 62: nozzle portion
    3, 33, 53, 63: fitting portion
    4, 34, 54: flange portion
    5: anticoagulant
    7, 8: syringe
    15, 35: support
    20, 22: syringe main body
    20a, 22a: distal portion
    21, 23: plunger
    22b: needle locking portion
    36, 43: outer wall projection portion
    38: dispensing kit
    39: dispensing container
    40: lid of dispensing container
    40a: opening of lid
    S: blood

Sa: blood remaining in distal end of nozzle portion

**Claims**

1. An adapter for blood sample dispensing which is mounted on a distal portion of a syringe and has a piping structure,

   wherein the adapter has a fitting portion fitted to the distal portion at one end, a nozzle portion at the other end, and a flange portion on an outer circumference surface, and
   wherein the adapter has a medicine, to be mixed in the blood sample, on the inside of the nozzle portion.

2. The adapter according to claim 1,

   wherein the adapter has an inner wall projection portion, which is formed in a spiral shape, on the inner wall surface of the nozzle portion.

3. The adapter according to claim 1 or 2,

   wherein the medicine is applied to the inner wall of the nozzle portion.

4. The adapter according to claim 1 or 2,

   wherein a support supporting the medicine is provided inside the nozzle portion.

5. The adapter according to any one of claims 1 to 4,

   wherein the flange portion has a rolling prevention portion.

6. The adapter according to claim 5,

   wherein the rolling prevention portion is a linear outer circumference portion which is provided in the outer circumference of the flange portion.

7. The adapter according to any one of claims 1 to 6,

   wherein an outer wall projection portion is erected on the outer wall surface of the nozzle portion, and a gap is caused between the flange portion and an opening portion by bringing the outer wall projection portion and a dispensing container into contact with each other when the nozzle portion is inserted into the opening portion which is formed in the dispensing container into which the blood sample is dispensed.

8. The adapter according to claim 7,

   wherein, in a cross-sectional view of the nozzle portion, the effective diameter of a distal portion of the nozzle portion, which is an effective diameter of the nozzle portion and a diameter of a minimum circumscribed circle of the nozzle portion and the outer wall projection portion, is a size allowing the distal portion to be inserted into the opening portion.

9. The adapter according to claim 8,

   wherein the outer wall projection portion has a tapered shape which tapers.

10. The adapter according to claim 8 or 9,

   wherein the outer wall projection portion extends in an axial direction of the nozzle portion.

11. The adapter according to claim 10,

   wherein the outer wall projection portion has a step portion in which the effective diameter of the nozzle portion changes from a value greater than the inner diameter of the opening portion to a value which allows the adapter to be fitted to the opening portion, toward a distal end.

12. The adapter according to claim 10 or 11,

   wherein there are three or more outer wall projection portions which are disposed in parallel in a circumferential direction of the nozzle portion.

13. The adapter according to claim 8 or 9,

   wherein the outer wall projection portion extends in a spiral shape along the circumferential surface of the nozzle portion.

14. The adapter according to any one of claims 8 to 13,

   wherein, when the inner diameter of the opening portion is set to W, the depth of the opening portion is set to D, the outer diameter of the distal end of the nozzle portion is set to $\phi 1$, and the effective diameter of the nozzle portion in a section, in which a gap caused due to the difference between the inner diameter of the opening portion and the effective diameter of the nozzle portion is formed between the opening portion and the section, out of sections of the nozzle portion and the outer wall projection portion, is set to $\phi 2$, the length L between a distal end of the outer wall projection portion and the distal end of the nozzle portion satisfies the following Formula.

$$\frac{\phi 1}{2} \cdot \frac{W - \phi 2}{D} \leq L < D$$

15. The adapter according to claim 7,

   wherein the effective diameter of a distal portion of the nozzle portion, which is an effective diameter of the nozzle portion and a diameter of a minimum circumscribed circle of the nozzle portion and the outer wall projection portion, is larger than the inner diameter of the opening portion.

16. The adapter according to any one of claims 1 to 6,

   wherein the flange portion has a through port or a notch.

17. The adapter according to any one of claims 1 to 16,

   wherein the length of the nozzle portion is 3 mm to 30 mm.

18. The adapter according to any one of claims 1 to 17,

   wherein the outer diameter of the flange portion is 8 mm to 30 mm.

19. The adapter according to any one of claims 1 to 18,

   wherein the volume of an internal space of the nozzle portion is 10 $\mu$L to 1 mL.

20. The adapter according to any one of claims 1 to 19,

   wherein the adapter has a specific color corresponding to the type of the medicine.

21. A dispensing kit comprising:

   the adapter according to any one of claims 8 to 14; and
   a dispensing container having an opening portion which is fitted to the outer wall projection portion of the adapter.

22. A needle kit comprising:

   the adapter according to any one of claims 1 to 20; and
   a needle having a needle base which is fitted to the distal portion of the syringe,
   wherein the nozzle portion of the adapter also capable of being fitted to the needle base.

23. The needle kit according to claim 22, comprising:

the syringe having the distal portion which is fitted to the fitting portion of the adapter.

# FIG. 1

(a)

(b)

(c)

# FIG. 2A

# FIG. 2B

# FIG. 3

# FIG. 4

# FIG. 5

## FIG. 6

(a)

17

17a

17b

17d

Y

Y

17c

(b)

17b

17d

17a

## FIG. 7

18b

18a

18

FIG. 8A

FIG. 8B

(b)

X

X

(c)

FIG. 9A

FIG. 9B

FIG. 9C

FIG. 9D

## FIG. 10A

## FIG. 10B

## FIG. 10C

## FIG. 10D

FIG. 11A

FIG. 11B

FIG. 11C

FIG. 12A

FIG. 12B

FIG. 13A    FIG. 13B    FIG. 13C

FIG. 14

FIG. 15A

FIG. 15B

FIG. 15C

FIG. 15D

FIG. 16

FIG. 17A                    FIG. 17B

FIG. 18A

FIG. 18B

FIG. 18C

## INTERNATIONAL SEARCH REPORT

| | International application No. |
|---|---|
| | PCT/JP2014/067535 |

**A. CLASSIFICATION OF SUBJECT MATTER**
*G01N1/00*(2006.01)i

According to International Patent Classification (IPC) or to both national classification and IPC

**B. FIELDS SEARCHED**

Minimum documentation searched (classification system followed by classification symbols)
G01N1/00-1/44

Documentation searched other than minimum documentation to the extent that such documents are included in the fields searched
Jitsuyo Shinan Koho        1922–1996   Jitsuyo Shinan Toroku Koho   1996–2014
Kokai Jitsuyo Shinan Koho  1971–2014   Toroku Jitsuyo Shinan Koho   1994–2014

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2013-47680 A (BREWER, William), 07 March 2013 (07.03.2013), | 1-6,16-20, 22-23 |
| A | claims 2, 20; paragraph [0036] & US 2010/0081209 A1   & EP 2125224 A1 & WO 2008/103828 A1 | 7-15,21 |
| Y | JP 2004-37446 A (Becton, Dickinson and Co.), 05 February 2004 (05.02.2004), paragraphs [0022], [0023]; fig. 2 & US 2004/0149015 A1   & EP 1338339 A1 & EP 1588766 A1         & DE 60308571 D & DE 60308571 T | 1-6,16-20, 22-23 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 September, 2014 (12.09.14) | 30 September, 2014 (30.09.14) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

International application No.

PCT/JP2014/067535

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 8-320274 A  (Precision System Science Co., Ltd.),<br>03 December 1996 (03.12.1996),<br>paragraphs [0073] to [0078]<br>& US 5895631 A          & US 2002/0123156 A1<br>& EP 763739 A1          & EP 1519196 A2<br>& WO 1996/029602 A1     & DE 69634794 D<br>& DE 69634794 T         & KR 10-0211129 B | 1-6,16-20, 22-23 |
| Y | JP 8-75725 A  (Eiken Chemical Co., Ltd.),<br>22 March 1996 (22.03.1996),<br>paragraph [0032]<br>& US 5514341 A          & EP 638803 A1<br>& WO 1994/015212 A1     & DE 69330461 D<br>& DE 69330461 T         & CN 1094164 A | 5-6,16 |

Form PCT/ISA/210 (continuation of second sheet) (July 2009)

**EP 3 018 464 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 58038536 A
- JP 2001224575 A